# EUROPÄISCHE PATENTSCHRIFT

(11) **EP 2 681 202 B1**
(45) Veröffentlichungstag und Bekanntmachung des Hinweises auf die Patenterteilung: **22.10.2014**
(21) Anmeldenummer: 12706057.2
(22) Anmeldetag: 29.02.2012
(51) Int. Cl.: C07D 307/68

(54) **VERFAHREN ZUR HERSTELLUNG VON NATRIUM- ODER KALIUMSALZEN DER 4-HYDROXY-2-OXO-2,5-DIHYDROFURAN-3-CARBONSÄUREESTER**
PROCESS FOR PREPARING SODIUM SALTS OR POTASSIUM SALTS OF 4-HYDROXY-2-OXO-2,5-DIHYDROFURAN-3-CARBOXYLATE
PROCÉDÉ DE PRODUCTION DE SELS DE SODIUM OU DE POTASSIUM DES ESTERS D'ACIDES 4-HYDROXY-2-OXO-2,5-DIHYDROFURAN-3-CARBOXYLIQUES

(30) Priorität: 03.03.2011 EP 11156806; 03.03.2011 US 201161448805 P
(43) Veröffentlichungstag der Anmeldung: 08.01.2014
(73) Patentinhaber: Bayer Intellectual Property GmbH, 40789 Monheim am Rhein (DE)
(72) Erfinder: FUNKE, Christian, 42799 Leichlingen (DE); FARIDA, Taraneh, 50259 Pulheim-Geyen (DE); BEECK, Stefan, 40668 Meerbusch (DE); LUI, Norbert, 51519 Odenthal (DE); MAIWALD, Berndt, 51399 Burscheid (DE)
(74) Vertreter: BIP Patents
(86) Internationale Anmeldenummer: PCT/EP2012/053418
(87) Internationale Veröffentlichungsnummer: WO 2012/117015

(56) Entgegenhaltungen:
- WO-A1-2009/036899
- BRESLOW D.S. ET AL.: "A New Synthesis of beta-Keto Esters of the Type RCOCH2COOC2H5", JOURNAL OF THE AMERICAN CHEMICAL SOCIETY, Bd. 66, 1944, Seiten 1286-1288, XP002633750, DOI: 10.1021/ja01236a022 in der Anmeldung erwähnt
- CAMPBELL A C ET AL: "SYNTHESIS OF (E)- AND (Z)-PULVINONES", JOURNAL OF THE CHEMICAL SOCIETY, PERKIN TRANSACTIONS 1, CHEMICAL SOCIETY, LETCHWORTH; GB, Bd. 1, 1. Januar 1985 (1985-01-01), Seiten 1567-1576, XP001005573, ISSN: 0300-922X, DOI: DOI:10.1039/P19850001567 in der Anmeldung erwähnt

## Beschreibung

Die vorliegende Erfindung betrifft ein Verfahren zur Herstellung von Natrium- oder Kaliumsalzen der 4-Hydroxy-2-oxo-2,5-dihydrofuran-3-carbonsäureester ausgehend von Malonsäureestern.

Die Herstellung von Hydroxy-2-oxo-2,5-dihydrofuran-3-carbonsäureester, der entsprechenden Tautomere, bzw. von deren Alkalimetallsalzen und auch deren Verwendung als Baustein in der Synthese biologisch aktiver Verbindungen ist bekannt (WO 2011/018180, WO 2009/036899, J. Chem. Soc., Perkin Trans. 1, 1985, Seiten 1567 bis 1576, Berichte der Deutschen Chemischen Gesellschaft (1911), 44, 1759 - 1765). Die bekannten Verfahren haben jedoch Nachteile wie nachfolgend beschrieben.

WO 2011/018180 beschreibt die Herstellung von Hydroxy-2-oxo-2,5-dihydrofuran-3-carbonsäureester und geht in der dort beschriebenen Synthese von Malonsäureester aus. Dieser wird mit einer Halogenacetylchloridverbindung in Gegenwart einer Base umgesetzt (siehe Reaktionsschema 1). Nach Zugabe von Wasser wird der gewünschte 4-Hydroxy-2-oxo-2,5-dihydrofuran-3-carbonsäureester erhalten. Die Base ist so gewählt, dass sie den Malonsäureester zu deprotonieren vermag, wodurch das Enolat des Malonsäureesters entsteht, das dann durch die Halogenacetylchloridverbindung acetyliert wird. Geeignete Basen sind insbesondere Alkoholate der allgemeinen Formel M(OR^{a})_{b}, in denen M für Na⁺, K⁺, Mg²⁺, b für 1 oder 2 steht und R^{a} für Methyl oder Ethyl steht. Als bevorzugt wird Natriummethylat genannt. Nach erfolgtem Ringschluss wird das gewünschte Produkt zusammen mit einem anorganischen Salz, das als Nebenprodukt anfällt, erhalten (z.B. NaCl, falls ein Natriumalkoholat als Base eingesetzt wird).

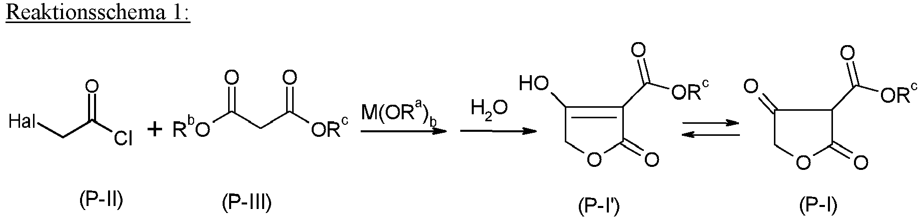

Die Abtrennung des anorganischen Salzes von der Reaktionsmischung, insbesondere wenn es sich um NaCl handelt, ist, wenn überhaupt, nur durch einen sehr großen technischen Aufwand zu erreichen, da die Verbindungen der Formel (P-I) und (P-I') sehr gut wasserlöslich sind. Eine Destillation ist nicht möglich, da sich die Verbindungen der Formel (P-I) und (P-I') bei höheren Temperaturen unter Freiwerden von CO₂ zersetzen. Das anorganische Salz wird deshalb nicht abgetrennt. Es wird in die nachfolgende Reaktion mit eingebracht und kann erst nach erfolgter Weiterreaktion der Verbindungen der Formel (P-I) und/oder (P-I') abgetrennt werden.

WO 2009/036899 beschreibt die Synthese von Salzen der 4-Hydroxy-2-oxo-2,5-dihydrofuran-3-carbonsäureester, das von Malonsäureester-Kaliumsalz ausgeht und in der unter Verwendung von Chloressigsäureester und einer Alkoholatbase, z.B. Natriummethylat, das entsprechende Salz des 4-Hydroxy-2-oxo-2,5-dihydrofuran-3-carbonsäureesters hergestellt wird (siehe Reaktionsschema 2). Bei dieser Reaktion entstehen keine anorganischen Salze, die mitgeschleppt werden müssen, jedoch verwendet die Reaktion Dimethylformamid bzw. Dimethylacetamid als Lösungsmittel, die zum Einen teuer sind und zum Anderen aufwändig entfernt und nur aufwändig zurück gewonnen werden können.

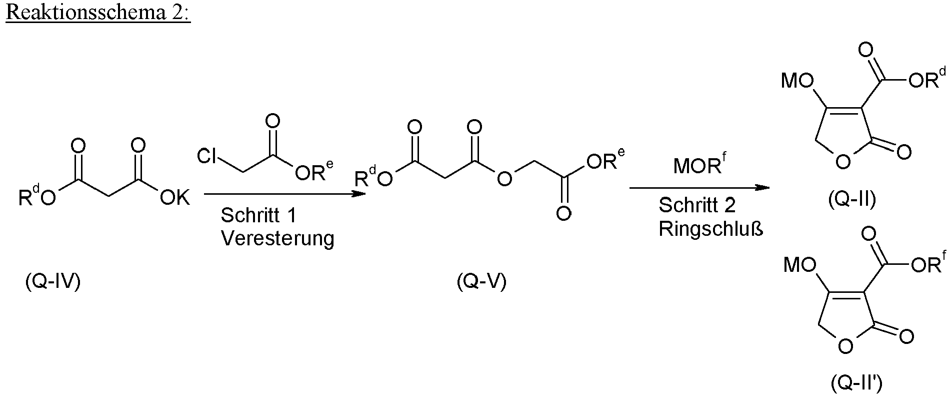

Schritt 1 der vorgenannten Reaktion stellt eine Veresterung dar, in der als Nebenprodukt Kaliumchlorid anfällt, das im weiteren von der Reaktionsmischung abgetrennt werden muss. In Schritt 2 findet der Ringschluss zur gewünschten Verbindung (Q-II) statt, wobei es zu einer Umesterung kommen kann, wodurch eine Verbindung der Formel (Q-II') isoliert wird. Die Umesterung im Schritt 2 kann dadurch, dass Alkoholate verwendet werden, die sterisch anspruchsvolle Reste R^{f} beinhalten (z.B. einen tert-Butylalkoholat), unterdrückt werden. Verbindungen der Formel (Q-IV) sind Feststoffe, kommerziell erhältlich oder können nach bekannten Verfahren hergestellt werden (vgl. J. Am. Chem. Soc. 1944, Nr. 66, Seite 1286, EP-A-950653, WO 2008/150487).

Die Reaktion ist für den großtechnischen Einsatz nachteilig, da sie von teuren, als Feststoff vorliegenden, Malonsäureester-Kaliumsalzen der Formel (Q-IV) ausgeht. Bei einer großtechnischen Herstellung ist das Verwenden von Feststoffen als Ausgangsmaterialien grundsätzlich unerwünscht, da die technische Handhabung solcher Feststoffe schwierig ist und es öfter zu einem Wechsel von Lösungsmitteln kommt, was insgesamt zu einem erheblichen technischen Aufwand bei der Reaktionsdurchführung führt.

Ausgehend von den bekannten Verfahren zur Herstellung von Salzen der 4-Hydroxy-2-oxo-2,5-dihydrofuran-3-carbonsäureester bzw. den entsprechenden Enol-Tautomeren stellt sich nun die Aufgabe, wie diese einfach und kostengünstig hergestellt werden können, so dass das Verfahren auch zur großtechnischen Herstellung der gewünschten Verbindung verwendet werden kann. Wünschenswert wäre es auch, ein Verfahren zu finden, das zumindest teilweise kontinuierlich abläuft und in dem aufwändige Isolierungen der Zwischenstufen vermieden werden. Unter kostengünstigen Verfahren werden solche Verfahren verstanden, die ohne großen finanziellen Aufwand durchzuführen sind, weil die Ausgangsstoffe beispielsweise kostengünstig und/oder ungefährlich sind, das Verfahren mit wenigen Verfahrensschritten auskommt bzw. sogar als "Eintopfreaktion" (d.h. ohne dass eine Isolierung von Zwischenstufen notwendig ist) durchzuführen ist, und/oder das gewünschte Natrium- oder Kaliumsalz der 4-Hydroxy-2-oxo-2,5-dihydrofuran-3-carbonsäureester in einer ausreichend hohen Ausbeute und Reinheit erhalten wird. Ferner ist es von Vorteil, ein Verfahren bereitzustellen, das Ressourcen schonend ist, in dem es z.B. weniger Energie verbraucht und/oder selektiv ist, d.h. dass nur im geringen Maße Nebenprodukte entstehen.

Es wurde nun ein Verfahren zur Herstellung von Natrium- oder Kaliumsalzen der 4-Hydroxy-2-oxo-2,5-dihydrofuran-3-carbonsäureester der allgemeinen Formel (I) gefunden, das die vorgenannten Nachteile vermeidet und das einfach und kostengünstig durchführbar ist, insbesondere weil es ohne aufwändige Isolierung und/oder Aufreinigung von Zwischenstufen auskommt. Auch wird kein Feststoff als Ausgangsmaterial verwendet, und es kann auf teure und aufwändige Lösungsmittel verzichtet werden. Das Verfahren kann sowohl kontinuierlich als auch diskontinuierlich ablaufen.

Es ist bekannt, dass Hydroxy-2-oxo-2,5-dihydrofuran-3-carbonsäureester auch als Tautomere vorliegen können, nämlich als 2,4-Dioxotetrahydrofuran-3-carbonsäureester. Jede Bezugnahme auf Hydroxy-2-oxo-2,5-dihydrofuran-3-carbonsäureester umfasst auch das entsprechende Tautomer.

Gegenstand der Erfindung ist somit das unten beschriebene Verfahren zur Herstellung von Natrium- oder Kaliumsalzen der 4-Hydroxy-2-oxo-2,5-dihydrofuran-3-carbonsäureester der Formel (I) 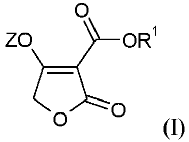 in dem Z für Natrium oder Kalium steht und R¹ die unten angegebene Bedeutung hat und die, wie bereits beschrieben, in den folgenden tautomeren Formen vorliegen kann:

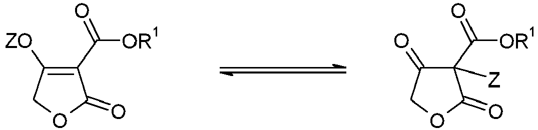

Die Anmeldung betrifft demzufolge ein Verfahren zur Herstellung von Natrium- oder Kaliumsalzen von 4-Hydroxy-2-oxo-2,5-dihydrofuran-3-carbonsäureester der Formel (I), umfassend die folgenden Schritte:

### Schritt (i):

Umsetzen eines symmetrischen Malonsäureesters der Formel (II) 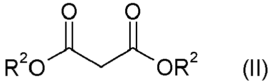 mit Kaliumhydroxid, das bevorzugt als wässrige Kaliumhydroxidlösung eingesetzt wird,
zum entsprechenden Malonsäureester-Kaliumsalz der Formel (III) 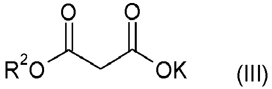 worin in den Formeln (II) und (III)
- R²: für C₁-C₁₂-Alkyl steht, bevorzugt steht R² für C₁-C₄-Alkyl, besonders bevorzugt steht R² für Methyl oder Ethyl,
in einem Lösungsmittel, vorzugsweise einem Alkohol, insbesondere Methanol oder Ethanol;

### Schritt (ii):

Umsetzen des Malonsäureester-Kaliumsalzes der Formel (III) mit einem Chloressigsäureester der Formel (IV) 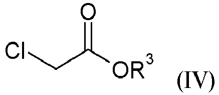 worin
- R³: für C₁-C₁₂-Alkyl steht, bevorzugt steht R³ für C₁-C₄-Alkyl, besonders bevorzugt steht R³ für Methyl oder Ethyl,
zu einer Verbindung der Formel (V) 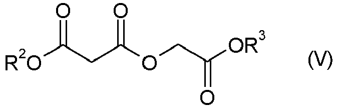 worin
- R²: die in Bezug auf Formel (III) aufgeführten Bedeutungen hat, und
- R³: die in Bezug auf Formel (IV) aufgeführten Bedeutungen hat,

### Schritt (iii) Ringschlussreaktion:

Umsetzen der Verbindung der Formel (V) mit einem Natrium- oder Kaliumalkoholat der Formel ZOR¹, worin
- R¹: für C₁-C₁₂-Alkyl steht, bevorzugt steht R¹ für C₁-C₆-Alkyl, besonders bevorzugt steht R¹ für Ethyl oder Methyl, und
- Z: für Natrium oder Kalium steht, bevorzugt für Natrium steht,
wobei nach Zugabe des Chloressigsäureesters in Schritt (ii) die Reaktionsmischung einer azeotropen Entwässerung unterzogen wird.

Das in Schritt (i) hergestellte Malonsäureester-Kaliumsalz der Formel (III) kann direkt vom Malonester der Formel (II) abgetrennt werden, vorzugsweise durch Phasentrennung, wobei das Malonsäureester-Kaliumsalz der Formel (III) in der wässrigen Phase und der Malonsäureester in der organischen Phase verbleibt.

Das Malonsäureester-Kaliumsalz wird in Schritt (i) in einer solchen Reinheit erhalten, dass es nach Abtrennung vom Reaktionsgemisch ohne weitere Aufreinigungsschritte in Schritt (ii) eingesetzt werden kann. Die Verbindung wird somit nicht als Feststoff, sondern gelöst eingesetzt. Erfindungsgemäß wird das noch aus Schritt (i) vorhandene Wasser vom Malonsäureester-Kaliumsalz der Formel (I) durch azeotrope Entwässerung der wässrigen Phase, enthaltend das Malonsäureester-Kaliumsalz der Formel (III), nach Zugabe des Chloressigsäureesters der Formel (IV) entfernt. Dadurch erfolgt die Umsetzung in Verfahrensschritt (ii) in Substanz.

Unter azeotroper Entwässerung wird hier die Abtrennung von Wasser durch azeotrope Destillation, vorzugsweise unter Zuhilfenahme eines Phasenabscheiders und in Gegenwart eines geeigneten Schleppmittels, das mit Wasser eine azeotrope Mischung ausbildet wie z.B. Toluol oder Xylol, verstanden.

Nach Abtrennung steht die nicht umgesetzte Verbindung der Formel (II) ohne weitere Aufarbeitung für eine erneute Reaktion mit Kaliumhydroxid zur Verfügung, was für das kontinuierliche Arbeiten einen großen Vorteil darstellt.

Das erfindungsgemäße Verfahren hat gegenüber dem in WO 2009/036899 beschriebenen Verfahren den Vorteil, dass bei der Umsetzung auf teure und schwierig zu entfernende und zurückzugewinnende Lösungsmittel, wie Dimethylformamid oder Dimethylacetamid verzichtet werden kann, und dass zu keiner Zeit das Malonsäureester-Kaliumsalz in fester Form isoliert wird.

Schritt (ii) wird vorzugsweise in Anwesenheit einer geringen (katalytischen) Menge an Wasser als Reaktionsvermittler durchgeführt. Unter geringen Mengen an Wasser werden, bezogen auf das Gesamtgewicht der Reaktionsmischung nach der azeotropen Entwässerung, folgende Mengen an Wasser verstanden: etwa 0,8 bis etwa 5,0 Gewichtsprozente (Gew.-%). Erfindungsgemäß bevorzugte Mengen sind etwa 0,8 bis etwa 4,0 Gew.-%, etwa 0,8 bis etwa 3,0 Gew.-%, etwa 0,8 bis etwa 2,0 Gew.-%, und etwa 0,8 bis etwa 1,5 Gew.-%.

Durch die Anwesenheit der geringen (katalytischen) Menge an Wasser wird die Reaktion in Schritt (ii) stark beschleunigt und zum vollständigen Umsatz gebracht.

Der in Schritt (ii) nicht umgesetzte Chloressigsäureester der Formel (IV) steht ohne weitere Aufarbeitung für eine erneute Reaktion mit dem Malonsäureester-Kaliumsalz der Formel (III) zur Verfügung, was für das kontinuierliche Arbeiten einen großen Vorteil darstellt.

Die in Schritt (ii) erhaltene Verbindung der Formel (V) wird in einer solchen Reinheit gewonnen, dass sie ohne Zwischenisolierung in Schritt (iii) eingesetzt werden kann. Es ist jedoch bevorzugt, die Verbindung der Formel (V) vor Weiterreaktion einer Destillation, Phasentrennung und einer azeotropen Trocknung zu unterziehen.

Geeignete, in Schritt (i) verwendbare Lösungsmittel sind Alkohole, insbesondere Methanol und Ethanol. Es können auch andere bekannte Alkohole eingesetzt werden. Das Lösungsmittel wird in einer solchen Menge eingesetzt, dass das Reaktionsgemisch während der Umsetzung gut rührbar bleibt. Vorteilhafterweise wird Schritt (i) in einer Mischung aus Alkohol und Wasser, insbesondere Methanol und Wasser oder Ethanol und Wasser durchgeführt. Gewöhnlicherweise wird das Wasser durch Verwenden einer wässrigen Kaliumhydroxidlösung in die Reaktionsmischung eingebracht. Sollte das so eingebrachte Wasser nicht reichen, kann selbstverständlich Wasser zur Reaktionsmischung hinzugefügt werden. Die Menge an zuzufügenden Wasser kann variieren und ist vom Fachmann leicht zu ermitteln.

Für die Ringschlussreaktion im Schritt (iii) kann jedes Natrium- oder Kaliumalkoholat verwendet werden, insbesondere Natriummethanolat oder Natriumethanolat. Aus technischen und ökonomischen Gründen ist es bevorzugt, Natrium- oder Kaliumalkoholate zu verwenden, die in dem entsprechenden Alkohol gelöst vorliegen und auch käuflich erhältlich sind (z.B. Natriummethanolat in Methanol gelöst), da dann kein weiteres Lösungsmittel verwendet werden muss.

Die Umsetzung des Schrittes (i), Schrittes (ii) und die Ringschlussreaktion des Schrittes (iii) kann bei Normaldruck, durchgeführt werden. Es ist auch möglich die Reaktion im Vakuum oder bei erhöhten Druck (Überdruck) durchzuführen.

Die Umsetzungen der Schritte (i), (ii) und (iii) laufen bei geeigneter Temperatur ab, die von den verwendeten Substraten abhängt. Geeignete Temperaturen lassen sich einfach durch Routineversuche ermitteln.

Schritt (i) kann beispielsweise bei einer Temperatur im Bereich von etwa -20°C bis etwa 20°C, vorzugsweise von etwa -10°C bis etwa 10°C durchgeführt werden. Besonders bevorzugt wird die Umsetzung bei einer Temperatur im Bereich von etwa -5°C bis etwa 0°C durchgeführt, bevorzugt unter Normaldruck.

Schritt (ii) kann beispielsweise bei einer Temperatur im Bereich von etwa 20°C bis etwa 200 °C, vorzugsweise von etwa 60°C bis etwa 150°C durchgeführt werden. Besonders bevorzugt wird die Umsetzung bei einer Temperatur im Bereich von etwa 80°C bis etwa 150°C durchgeführt, bevorzugt unter Normaldruck.

Schritt (iii) kann beispielsweise bei einer Temperatur im Bereich von etwa 20°C bis etwa 120°C, vorzugsweise von etwa 20°C bis etwa 100°C durchgeführt werden. Besonders bevorzugt wird die Umsetzung bei einer Temperatur im Bereich von etwa 20°C bis etwa 80°C durchgeführt, bevorzugt unter Normaldruck.

In den Schritten (i), (ii) und (iii) des erfindungsgemäßen Verfahren können die molaren Verhältnisse der Verbindung der Formel (II) und Kaliumhydroxid, bzw. der Verbindung der Formel (III) zur Verbindung der Formel (IV), oder der Verbindung der Formel (V) zum Alkalimetallalkoholat ZOR¹ in weiten Bereichen variieren. Sie unterliegen im Allgemeinen keiner Beschränkung.

In Schritt (i) ist es vorteilhaft, wenn das molare Verhältnis der Verbindung der Formel (II) zu Kaliumhydroxid im Bereich von etwa 1,1 bis etwa 10, insbesondere im Bereich von etwa 1,5 bis etwa 6 liegen. Erfindungsgemäß bevorzugt liegt das molare Verhältnis im Bereich von etwa 1,8 bis 3.

In Schritt (ii) ist es vorteilhaft, wenn das molare Verhältnis der Verbindung der Formel (IV) zur Verbindung der Formel (III) im Bereich von etwa 10 bis etwa 1, insbesondere im Bereich von etwa 8 bis etwa 1 liegen. Erfindungsgemäß bevorzugt liegt das molare Verhältnis im Bereich von etwa 6 bis 1.

In Schritt (iii) ist es vorteilhaft, wenn das molare Verhältnis von Verbindung der Formel (V) zum Alkalimetallalkoholat im Bereich von etwa 0,9 bis etwa 10, insbesondere im Bereich von etwa 1 bis etwa 5 liegen. Erfindungsgemäß bevorzugt liegt das molare Verhältnis im Bereich von etwa 1 bis 2.

Die Bezugnahme auf Alkyl umfasst verzweigte oder unverzweigte Alkyle. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste, speziell Methyl und Ethyl.

Die vorliegende Erfindung wird anhand der nachfolgenden Beispiele näher erläutert, wobei die Beispiele nicht in die Erfindung einschränkender Weise zu interpretieren sind.

### Herstellungsbeispiel:

### Beispiel 1:

### Herstellung von Natrium-4-(methoxycarbonyl)-5-oxo-2,5-dihydrofuran-3-olat und Natrium-4-(ethoxycarbonyl)-5-oxo-2,5-dihydrofuran-3-olat

Schritt (i): 481 g (3,00 mol) Malonsäurediethylester und 300 g Ethanol werden vorgelegt und auf -5°C temperiert. Zu dieser Mischung werden bei -5°C bis 0°C 337 g (1,50 mol) einer 25%-igen wässrigen KOH-Lösung in 2 Stunden eindosiert. Es wird 30 Minuten bei 0°C nachgerührt und anschließend bei 30°C Ethanol abdestilliert. Die beiden Phasen werden bei 5°C getrennt, wobei die obere organische Phase, in der sich der nicht umgesetzte Malonsäurediethylester befindet, direkt für eine weitere Verseifung verwendet werden kann.

Schritt (ii): Die wässrige Phase wird mit 458 g (3,75 mol) Chloressigsäureethylester im Vakuum azeotrop bei 30-50°C entwässert. Nach Zugabe von weiteren 458 g (3,75 mol) Chloressigsäureethylester und 9,4 g Wasser (0,8 Gewichtsprozente bezogen auf Masse der Reaktionsmischung) als Reaktionsvermittler wird dann auf 105°C erwärmt und 2,5 Stunden bei dieser Temperatur nachgerührt. Anschließend wird zunächst bei 60-80°C Chloressigsäurethylester abdestilliert und nach Zugabe von 375 g Wasser die restliche Menge an Chloressigsäureethylester azeotrop entfernt. Nach dem Abkühlen auf 20°C wird die wässrige Phase abgetrennt.

Schritt (iii): Die organische Phase wird mit 50 g Xylol bei 50-70°C im Vakuum azeotrop getrocknet und anschließend bei 40-50°C innerhalb von 1 Stunde mit 270 g (1,50 mol) einer 30%-igen Natriummethylat-Lösung in Methanol versetzt. Die Mischung wird danach für 2 Stunden auf 65°C erwärmt, anschließend auf 20°C abgekühlt, 1 Stunde bei dieser Temperatur verrührt und filtriert. Der Rückstand wird mit 60 g Methanol als Verdrängungswäsche gewaschen und im Vakuum getrocknet. Man erhält 209 g als 7:3 Mischung von Natrium-4-(methoxycarbonyl)-5-oxo-2,5-dihydrofuran-3-olat und Natrium-4-(ethoxycarbonyl)-5-oxo-2,5-dihydrofuran-3-olat. Bezogen auf eingesetztes Kaliumhydroxid entspricht das einer isolierten Ausbeute von 76 %.

| | | |
|---|---|---|
| ¹H-NMR (D₂O, 298K) | δ: 3,73 s (3H), 4,42 s (2H) | [Hauptkomponente]; |
| | 1,30 t (3H), 4,23 q (2H), 4,42 s (2H) | [Nebenkomponente] |

## Patentansprüche

1. Verfahren zur Herstellung von Natrium- oder Kaliumsalzen der 4-Hydroxy-2-oxo-2,5-dihydrofuran-3-carbonsäureester der Formel (I) 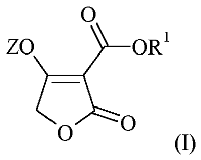 worin
Z für Natrium oder Kalium steht, und
R¹ für C₁-C₁₂-Alkyl steht,
umfassend die folgenden Schritte:
Schritt (i): Umsetzen eines symmetrischen Malonsäureesters der Formel (II) 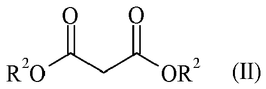 mit Kaliumhydroxid zum entsprechenden Malonsäureester-Kaliumsalz der Formel (III) 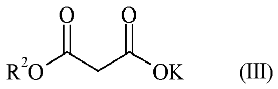 worin in den Formeln (II) und (III)
R² für C₁-C₁₂-Alkyl steht,
in einem Lösungsmittel;
Schritt (ii): Umsetzen des Malonsäureester-Kaliumsalzes der Formel (III) mit einem Chloressigsäureester der Formel (IV) 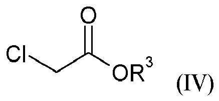
worin
R³ für C₁-C₁₂-Alkyl steht,
zu einer Verbindung der Formel (V) 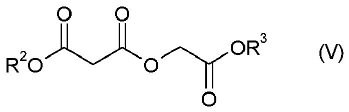 worin
R² die in Bezug auf Formel (III) aufgeführten Bedeutungen hat, und
R³ die in Bezug auf Formel (IV) aufgeführten Bedeutungen hat,
Schritt (iii): Umsetzen der Verbindung der Formel (V) mit einem Natrium- oder Kaliumalkoholat der Formel ZOR¹, worin
R¹ für C₁-C₁₂-Alkyl steht, und
Z für Natrium oder Kalium steht,
wobei nach Zugabe des Chloressigsäureesters in Schritt (ii) die Reaktionsmischung einer azeotropen Entwässerung unterzogen wird

2. Verfahren nach Anspruch 1, in dem in Schritt (i) eine wässrige Kaliumhydroxidlösung eingesetzt wird und worin das Lösungsmittel in Schritt (i) ein Alkohol ist.

3. Verfahren nach Anspruch 1 oder 2, in dem nach erfolgter Umsetzung des Schrittes (i) das Malonsäureester-Kaliumsalz der Formel (III) vom nicht umgesetzten Malonsäureester der Formel (II) über Phasentrennung abgetrennt wird, bevor es in Schritt (ii) eingesetzt wird.

4. Verfahren nach Anspruch 1, in dem in Schritt (ii) der Reaktionsmischung nach Durchführung einer azeotropen Entwässerung wieder 0,8 bis 5,0 Gew.-% Wasser zugegeben wird.

5. Verfahren nach Anspruch 4, in dem Schritt (ii) in Substanz durchgeführt wird.

6. Verfahren nach einem der Ansprüche 1 bis 5, in dem in Schritt (iii) Natriummethanolat, Natiumethanolat, Kaliummethanolat oder Kaliumethanolat verwendet wird.

7. Verfahren nach Anspruch 6, in dem das Natriummethanolat oder das Kaliummethanolat in Methanol gelöst vorliegt und das Natriumethanolat oder das Kaliumethanolat in Ethanol gelöst vorliegt.

## Claims

1. Process for preparing sodium or potassium salts of 4-hydroxy-2-oxo-2,5-dihydrofuran-3-carboxylic esters of the formula (I) 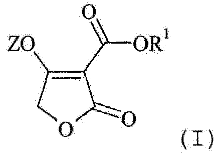 in which
Z is sodium or potassium, and
R1 is C1-C12-alkyl,
comprising the hollowing steps:
step (i) : reacting a symmetric malonic ester of the formula (II) 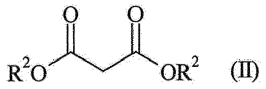 with potassium hydroxide to give the corresponding malonic ester potassium salt of the formula (III) 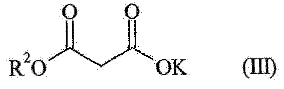 in which, in the formulae (II) and (III),
R2 is C1-C12-alkyl, in a solvent;
step (ii) : reacting the malonic ester potassium salt of the formula (III) with a chloroacetic ester of the formula (IV) 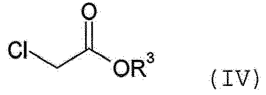 in which
R3 is C1-C12-alkyl, to give a compound of the formula (V) 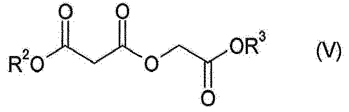 in which
R2 has the definitions listed in relation to formula (III), and
R3 has the definitions listed in relation to formula (IV),
step (iii) : reacting the compound of the formula (V) with a sodium or potassium alkoxide of the formula ZOR1 in which
R1 is C1-C12-alkyl, and
Z is sodium or potassium,
wherein, after addition of the chloroacetic ester in step (ii), the reaction mixture is subjected to azeotropic dewatering.

2. Process according to Claim 1, in which an aqueous potassium hydroxide solution is used in step (i) and in which the solvent in step (i) is an alcohol.

3. Process according to Claim 1 or 2, in which, on complexion of conversion in step (i), the malonic ester potassium salt of the formula (III) is separated from the unconverted masonic ester of the formula (II) by means of phase separation before it is used in step (ii).

4. Process according to Claim 1, in which, in step (ii), 0.8 to 5.0% by weight of water is added again to the reaction mixture after performance of an azeotropic dewatering.

5. Process according to Claim 4, in which step (ii) is performed in substance.

6. Process according to any of Claims 1 to 5, in which, in step (iii), sodium methoxide, sodium ethoxide, potassium methoxide or potassium ethoxide is used.

7. Process according two Claim 6, in which the sodium methoxide or the potassium methoxide is present dissolved in methanol, and the sodium ethoxide or the potassium ethoxide is present dissolved in ethanol.

## Revendications

1. Procédé pour la préparation de sels de sodium ou potassium des esters d'acide 4-hydroxy-2-oxo-2,5-dihydrofurane-3-carboxylique de formule (I), 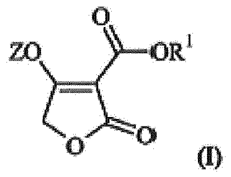 dans laquelle
Z représente le sodium ou le potassium, et
R représente un groupe alkyle en C₁-C₁₂,
comprenant les étapes suivantes :
étape (i) : mise en réaction d'un ester symétrique d'acide malonique de formule (II) 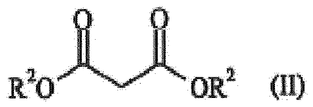 avec de l'hydroxyde de potassium, conduisant au sel de potassium d'ester d'acide malonique de formule (III) correspondant 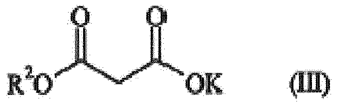 où dans les formules (II) et (III)
R² représente un groupe alkyle en C₁-C₁₂,
dans un solvant ;
étape (ii) : mise en réaction du sel de potassium d'ester d'acide malonique de formule (III) avec un ester d'acide chloracétique de formule (IV) 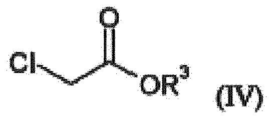 dans laquelle
R³ représente un groupe alkyle en C₁-C₁₂, conduisant à un composé de formule (V) 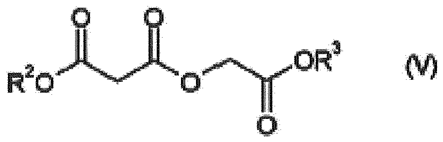 dans laquelle
R² a les significations indiquées au sujet de la formule (III), et
R³ a les significations indiquées au sujet de la formule (IV),
étape (iii) : mise en réaction du composé de formule (V) avec un alcoolate de sodium ou de potassium de formule ZOR¹, dans laquelle
R¹ représente un groupe alkyle en C₁-C₁₂, et
Z représente le sodium ou le potassium,
en soumettent dans l'étape (ii) le mélange réactionnel, après addition de l'ester d'acide chloracétique, à une déshydratation azéotropique.

2. Procédé selon la revendication 1, dans lequel on utilise dans l'étape (i) une solution aqueuse d'hydroxyde de potassium et dans lequel le solvant dans l'étape (i) est un alcool.

3. Procédé selon la revendication 1 ou 2, dans lequel une fois effectuée la réaction de l'étape (i) on sépare par séparation de phases le sel de potassium d'ester d'acide malonique de formule (III) d'avec l'ester diacide malonique de formule (II) n'ayant pas réagi, avant de l'utiliser dans l'étape (ii).

4. Procédé selon la revendication 1, dans lequel après avoir effectué une déshydratation azéotropique on ajoute au mélange réactionnel dans l'étape (ii) de nouveau 0,8 à 5,0 % en poids d'eau.

5. Procédé selon la revendication 4, dans lequel on procède sans soldant dans l'étape (ii).

6. Procédé selon l'une quelconque des revendications 1 à 5, dans lequel on utilise dans l'étape (iii) du méthanolate de sodium, de l'éthanolate de sodium, du méthanolate de potassium ou de l'éthanolate de potassium.

7. Procédé selon la revendication 6, dans lequel le méthanolate de sodium ou le méthanolate de potassium est présent en solution dans du méthanol et l'éthanolate de sodium ou l'éthanolate de potassium est présent en solution dans de l'éthanol.
